# EUROPEAN PATENT APPLICATION

(11) **EP 4 502 131 A1**
(43) Date of publication of application: **05.02.2025**
(21) Application number: 23795855.8
(22) Date of filing: 13.02.2023
(51) Int. Cl.: C12M 1/00, C12M 1/02

(54) **CULTURE APPARATUS**

(30) Priority: 26.04.2022 JP 2022072630
(71) Applicant: CANON KABUSHIKI KAISHA, Tokyo 146-8501 (JP)
(72) Inventor: HIGUCHI, Akira, Kyoto-shi, Kyoto 613-0916 (JP)
(74) Representative: WESER & Kollegen Patentanwälte PartmbB
(86) International application number: PCT/JP2023/004814
(87) International publication number: WO 2023/210107

(57) **Abstract**

Provided is a culture apparatus including: a culture vessel configured to accommodate a culture solution for culturing cells; a tilting device configured to enable tilting of the culture vessel in a plurality of different directions in top view; and a suction nozzle having a suction port through which the culture solution in the culture vessel is sucked, the suction port being arranged inside the culture vessel. The tilting device is configured to, when the culture solution in the culture vessel is sucked through the suction nozzle, tilt the culture vessel so that the culture solution has the largest liquid depth at a position far from a suction range for the suction port of the suction nozzle.

## Description

### [Technical Field]

The present disclosure relates to a culture apparatus for culturing cells with a culture solution.

### [Background Art]

Hitherto, a culture method of culturing cells while appropriately replacing a culture solution in a culture vessel has been put into practice. For example, in Patent Literature 1, there is disclosed a culture vessel having a sample inlet/outlet. A culture solution can be collected via the sample inlet/outlet by tilting the culture vessel.

### [Citation List]

### [Patent Literature]

PTL 1: International Publication No. WO2017/078007

### [Summary of Invention]

### [Technical Problem]

In the case of the culture vessel as disclosed in Patent Literature 1, however, turbulence may occur in the culture solution in the culture vessel at the time of collection of the culture solution, and stress may act on cells. As a result, there is a possibility that the cells may be damaged.

Thus, the present disclosure has an object to, in cell culture for culturing cells in a culture vessel with a cell solution, reduce stress on cells, which is caused when the culture solution is collected from the culture vessel or when the culture solution is supplied into the culture vessel.

### [Solution to Problem]

In order to achieve the above-mentioned technical object, according to one aspect of the present disclosure, there is provided a culture apparatus including: a culture vessel configured to accommodate a culture solution for culturing cells; a tilting device configured to enable tilting of the culture vessel in a plurality of different directions in top view; and a suction nozzle having a suction port through which the culture solution in the culture vessel is sucked, the suction port being arranged inside the culture vessel, wherein, the tilting device is configured to, when the culture solution in the culture vessel is sucked through the suction nozzle, tilt the culture vessel so that the culture solution has the largest liquid depth at a position far from a suction range for the suction port of the suction nozzle.

Further, according to another aspect of the present disclosure, there is provided a culture apparatus including: a culture vessel configured to accommodate a culture solution for culturing cells; a tilting device configured to enable tilting of the culture vessel in a plurality of different directions in top view; and a supply nozzle having a supply port through which the culture solution is supplied into the culture vessel, the supply port being arranged inside the culture vessel, wherein, the tilting device is configured to, when the culture solution is supplied into the culture vessel through the supply nozzle, tilt the culture vessel so that the culture solution has the largest liquid depth at a position far from the supply port of the supply nozzle.

### [Advantageous Effects of Invention]

According to the present disclosure, in the cell culture for culturing cells in the culture vessel with the cell solution, it is possible to reduce stress on cells, which is caused when the culture solution is collected from the culture vessel or when the culture solution is supplied into the culture vessel.

### [Brief Description of Drawings]

[FIG. 1]

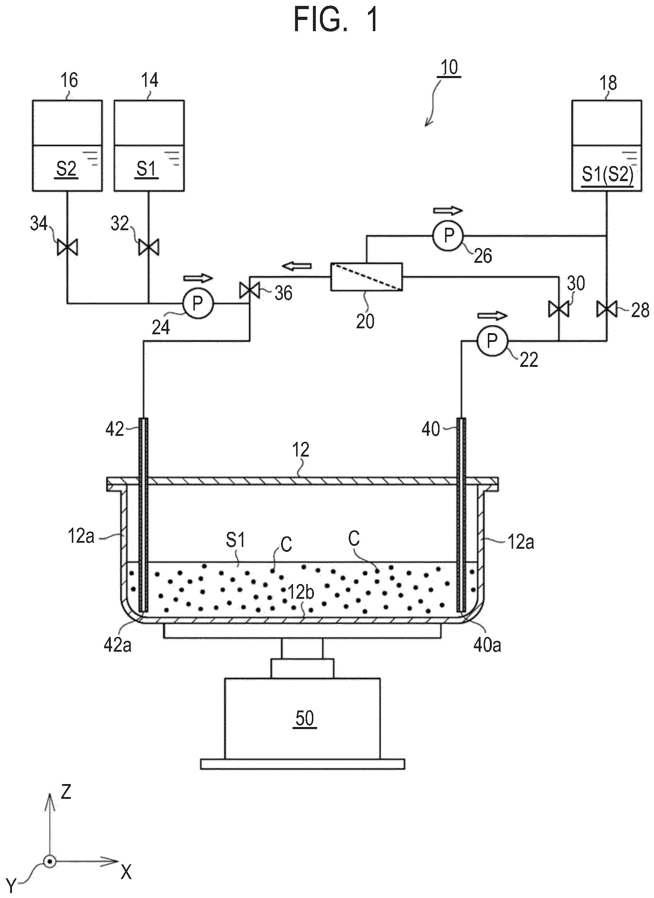

   FIG. 1 is a schematic view for illustrating a configuration of a culture apparatus according to one embodiment of the present disclosure.
[FIG. 2]

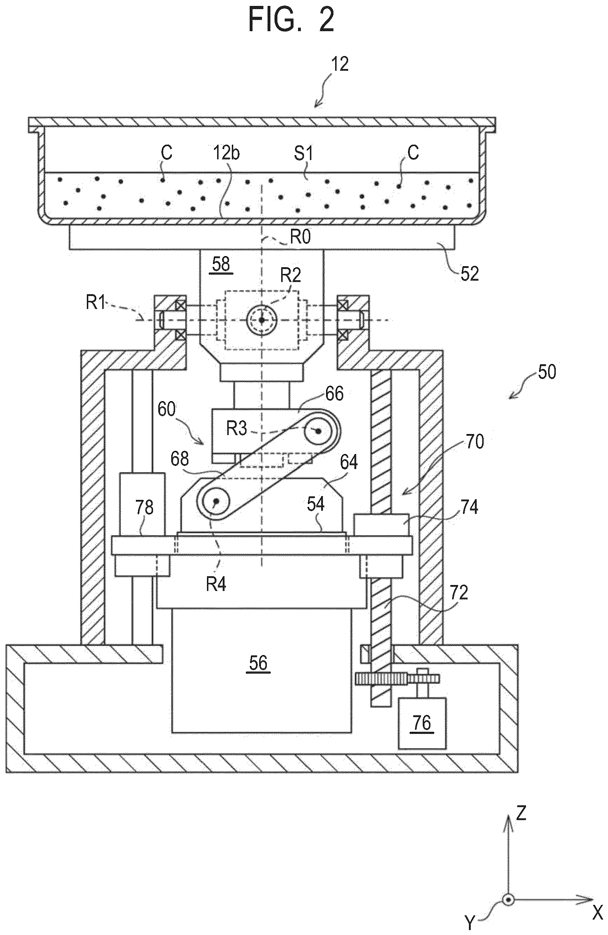

   FIG. 2 is a schematic partial sectional view of a tilting device of the culture apparatus.
[FIG. 3]

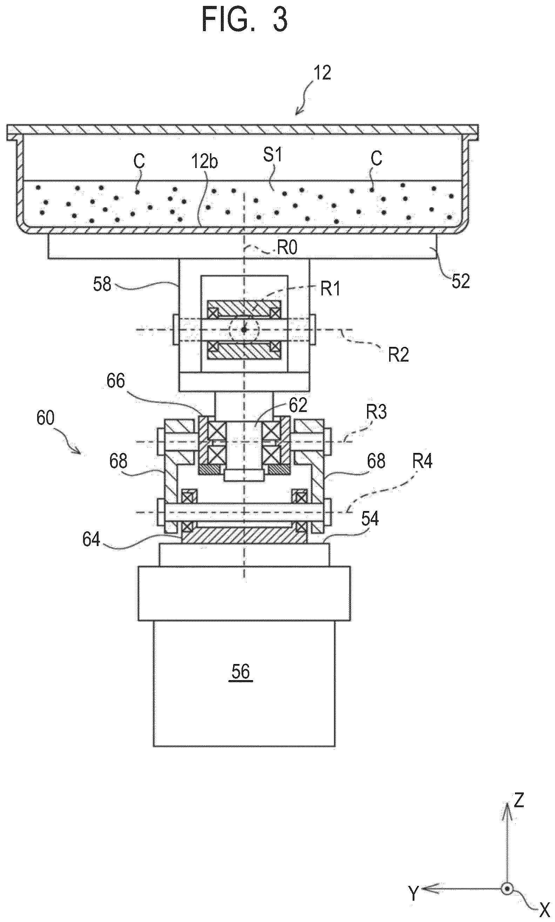

   FIG. 3 is a schematic partial sectional view of a part of the tilting device as viewed from a different direction.
[FIG. 4]

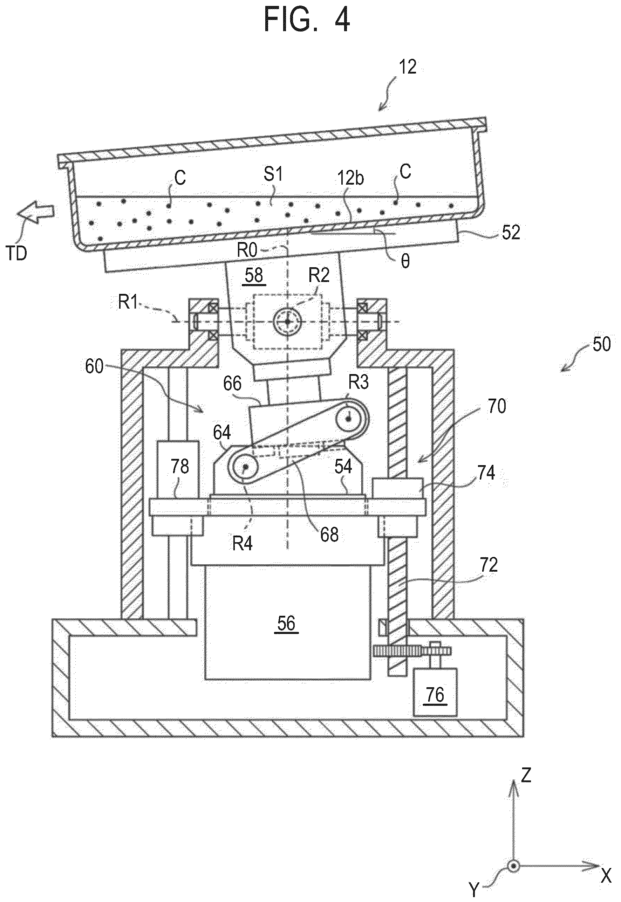

   FIG. 4 is a schematic partial sectional view of the tilting device in a state in which a stage is tilted.
[FIG. 5]

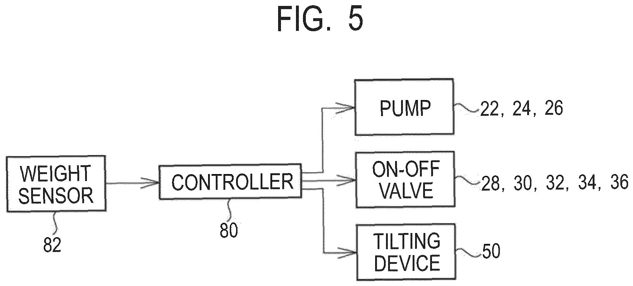

   FIG. 5 is a block diagram for illustrating a control system of the culture apparatus, which is associated with replacement of a culture solution in a culture vessel.
[FIG. 6A]

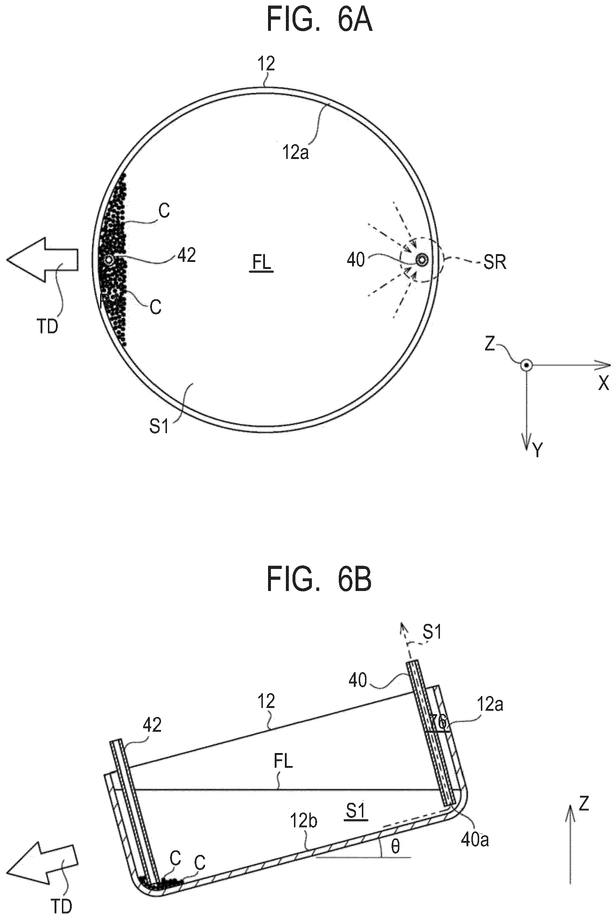

   FIG. 6A is a top view of the culture vessel during the replacement of the culture solution.
[FIG. 6B]
   FIG. 6B is a sectional view of the culture vessel during the replacement of the culture solution.
[FIG. 7A]

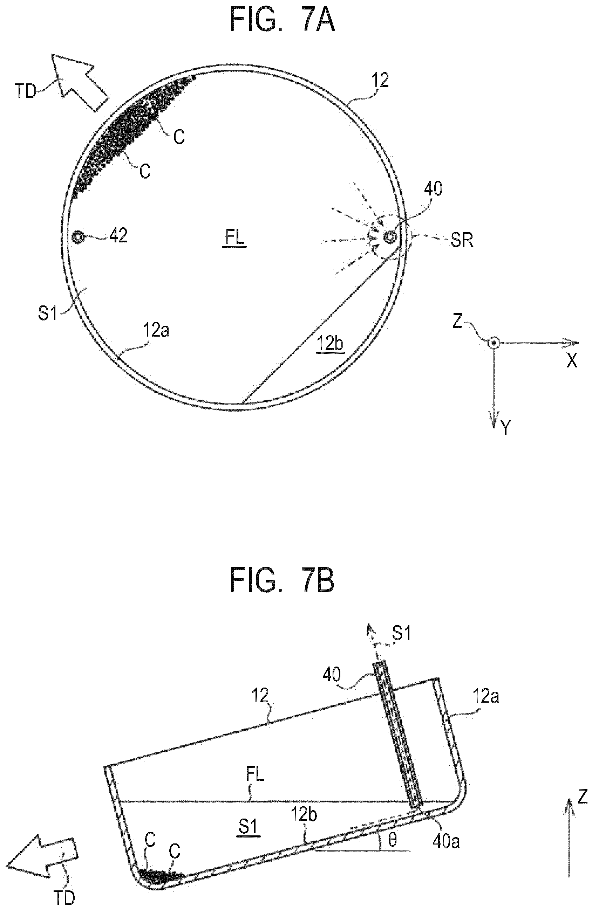

   FIG. 7A is a top view of the culture vessel during the replacement of the culture solution, following FIG. 6A.
[FIG. 7B]
   FIG. 7B is a sectional view of the culture vessel during the replacement of the culture solution, following FIG. 6B.
[FIG. 8A]

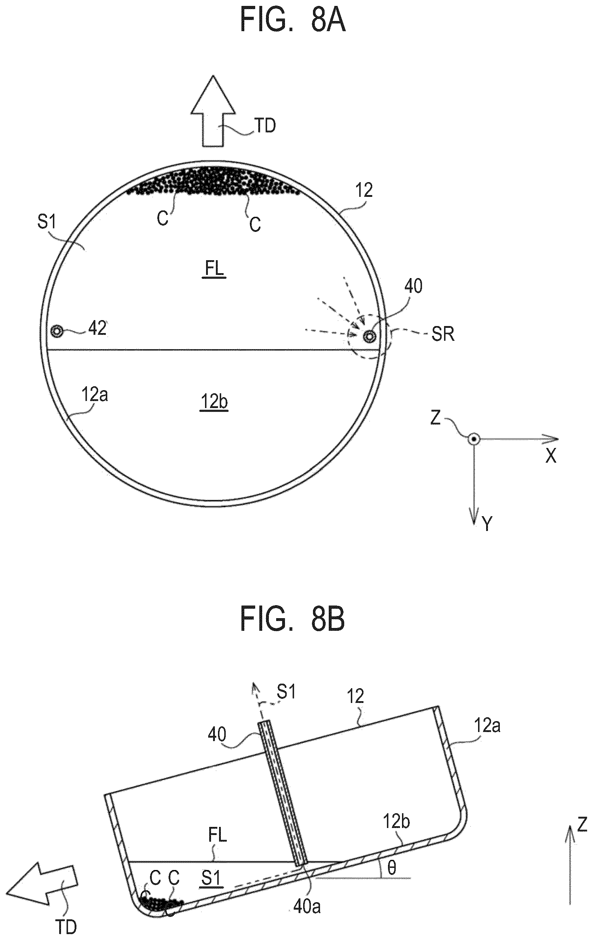

   FIG. 8A is a top view of the culture vessel during the replacement of the culture solution, following FIG. 7A.
[FIG. 8B]
   FIG. 8B is a sectional view of the culture vessel during the replacement of the culture solution, following FIG. 7B.
[FIG. 9A]

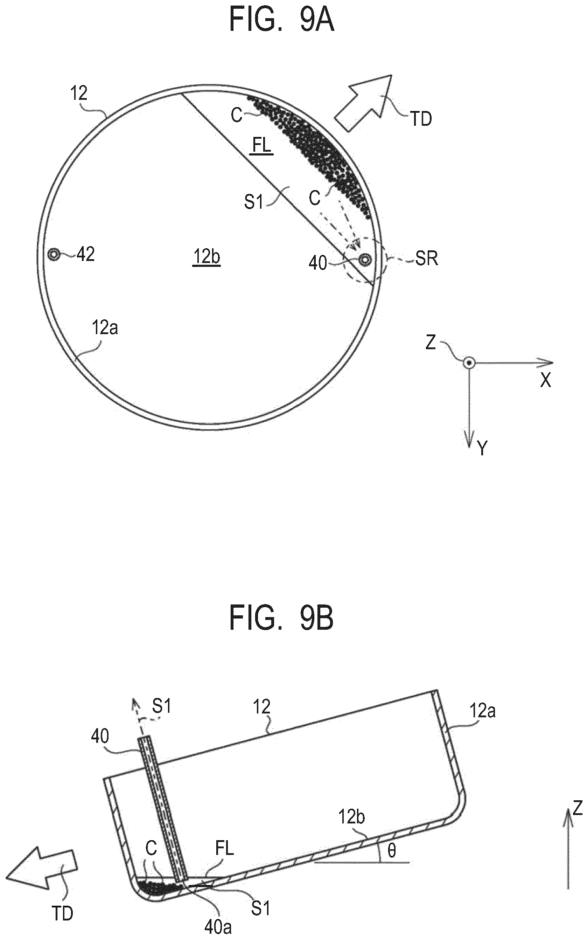

   FIG. 9A is a top view of the culture vessel during the replacement of the culture solution, following FIG. 8A.
[FIG. 9B]
   FIG. 9B is a sectional view of the culture vessel during the replacement of the culture solution, following FIG. 8B.
[FIG. 10A]

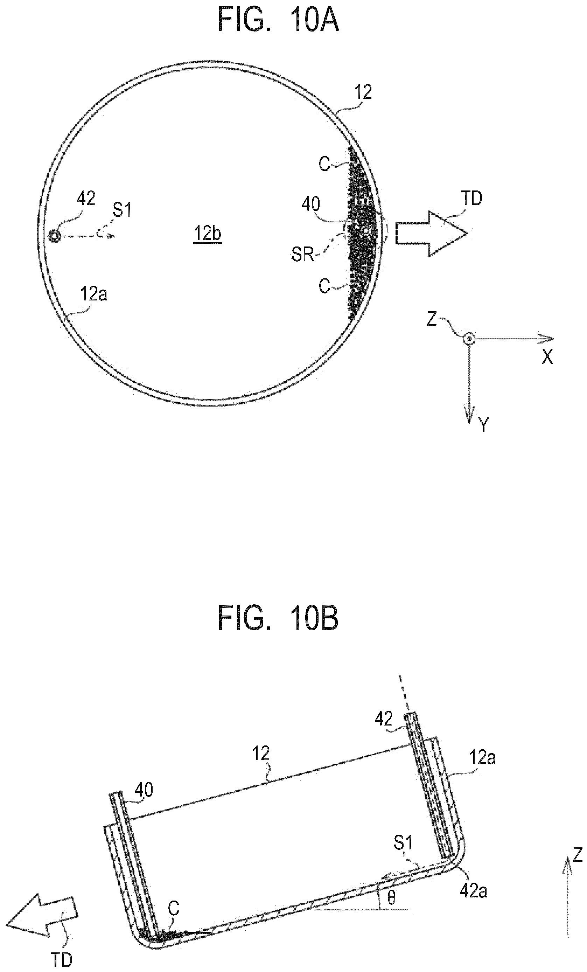

   FIG. 10A is a top view of the culture vessel during the replacement of the culture solution, following FIG. 9A.
[FIG. 10B]
   FIG. 10B is a sectional view of the culture vessel during the replacement of the culture solution, following FIG. 9B.
[FIG. 11]

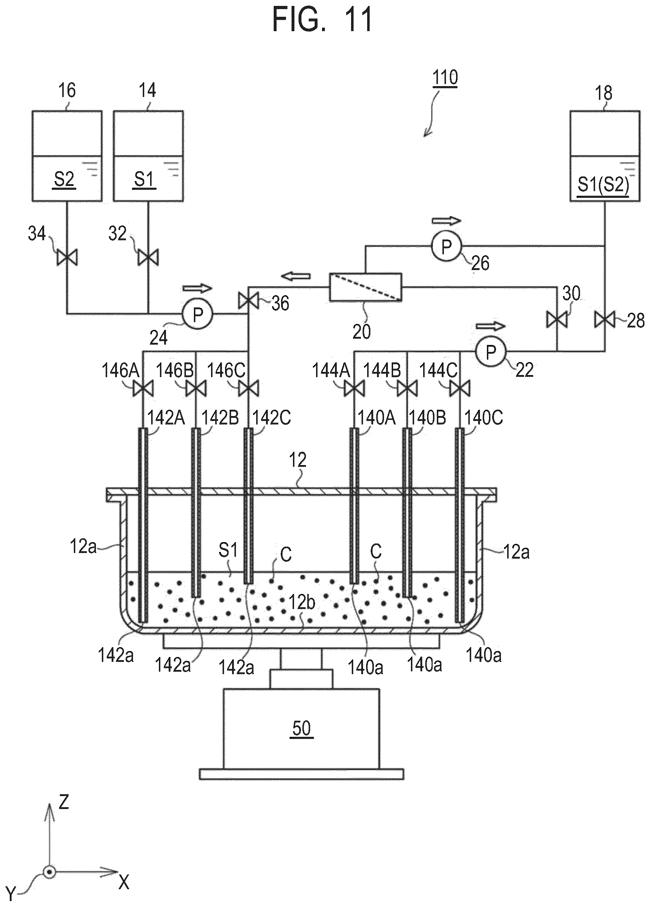

   FIG. 11 is a schematic view for illustrating a configuration of a culture apparatus according to another embodiment of the present disclosure.

### [Description of Embodiments]

A culture apparatus according to one aspect of the present disclosure includes: a culture vessel configured to accommodate a culture solution for culturing cells; a tilting device configured to enable tilting of the culture vessel in a plurality of different directions in top view; and a suction nozzle having a suction port through which the culture solution in the culture vessel is sucked, the suction port being arranged inside the culture vessel, wherein, the tilting device is configured to, when the culture solution in the culture vessel is sucked through the suction nozzle, tilt the culture vessel so that the culture solution has the largest liquid depth at a position far from a suction range for the suction port of the suction nozzle.

According to this aspect, in cell culture for culturing cells in the cell vessel with the cell solution, stress on the cells, which is caused when the culture solution is collected from the culture vessel away from the suction range for the suction port of the suction nozzle, can be reduced.

For example, the tilting device may be configured to change a tilt direction of the culture vessel so that the suction port remains positioned below a liquid level of the culture solution, the liquid level continuously falling due to suction through the suction nozzle.

For example, the culture apparatus may further include a weight sensor configured to measure weight of the culture vessel. In this case, the tilting device changes the tilt direction of the culture vessel based on a result of measurement performed by the weight sensor.

For example, the culture apparatus may further include a supply nozzle having a supply port through which a culture solution is supplied into the culture vessel, the supply port being arranged inside the culture vessel. In this case, when the culture solution is supplied into the culture vessel through the supply nozzle, the tilting device tilts the culture vessel so that the culture solution has the largest liquid depth at a position far from the supply port of the supply nozzle.

For example, after the culture solution in the culture vessel is sucked through the suction nozzle, the culture solution may be supplied into the suction vessel through the supply nozzle.

For example, the tilting device may be configured to tilt the culture vessel so that the culture solution has the largest liquid depth at a position inside the culture vessel, the position being the farthest from each of the suction nozzle and the supply nozzle, and the culture solution may be supplied into the culture vessel through the supply nozzle while the culture solution in the culture vessel is being sucked through the suction nozzle.

For example, the culture apparatus may further include a filter configured to filter only the culture solution sucked through the suction nozzle, and liquid containing the culture solution filtered through the filter may be supplied into the culture vessel from the supply nozzle.

For example, the culture apparatus may further include a weight sensor configured to measure weight of the culture vessel. In this case, a supply amount of the liquid containing the culture solution filtered through the filter into the culture vessel is adjusted so that a time rate of change of a measurement value obtained by the weight sensor falls within a predetermined range.

For example, the tilting device may include: a stage on which the culture vessel is to be placed; a rotary actuator including a rotary table configured to rotate about a rotation center axis extending in a vertical direction; a rocking head configured to support the stage and to be rockable about a first rocking axis extending in a horizontal direction and a second rocking axis extending in the horizontal direction and being orthogonal to the first rocking axis, the rocking head including a coupling shaft; a tilting mechanism including a rocking head coupling portion, which is slidably inserted over the coupling shaft of the rocking head to be coupled to the rocking head, a base portion mounted to the rotary table of the rotary actuator, and a link arm including one end turnably fixed to the rocking head coupling portion and another end turnably fixed to the base portion; and a rotary actuator raising/lowering mechanism configured to raise and lower the rotary actuator in the vertical direction.

Further, a culture apparatus according to another aspect of the present disclosure includes: a culture vessel configured to accommodate a culture solution for culturing cells; a tilting device configured to enable tilting of the culture vessel in a plurality of different directions in top view; and a supply nozzle having a supply port through which the culture solution is supplied into the culture vessel, the supply port being arranged inside the culture vessel, wherein, the tilting device is configured to, when the culture solution is supplied into the culture vessel through the supply nozzle, tilt the culture vessel so that the culture solution has the largest liquid depth at a position far from the supply port of the supply nozzle.

According to this aspect, in cell culture for culturing cells in the cell vessel with the cell solution, stress on the cells, which is caused when the culture solution is supplied into the culture vessel, can be reduced.

Hereinbelow, with reference to the drawings, description is given of embodiments of the present disclosure.

FIG. 1 is a schematic view for illustrating a configuration of a culture apparatus according to one embodiment of the present disclosure. An X-Y-Z orthogonal coordinate system shown in the drawings is for facilitating understanding of embodiments of the present disclosure, and does not limit the embodiments of the present disclosure. A Z axis direction indicates the vertical direction, and an X axis direction and a Y axis direction indicate the horizontal direction.

As illustrated in FIG. 1, a culture apparatus 10 includes a culture vessel 12 configured to accommodate a culture solution S1 containing cells C. Further, in the case of this embodiment, the culture apparatus 10 includes a culture solution supply vessel 14, a wash solution supply vessel 16, a liquid waste collection vessel 18, a hollow fiber membrane filter 20, a plurality of pumps 22, 24, and 26, and a plurality of on-off valves 28, 30, 32, 34, and 36. The culture solution supply vessel 14 stores a fresh culture solution S1 to be supplied into the culture vessel 12. The wash solution supply vessel 16 stores a wash solution S2 for washing the cells C in the culture vessel 12. The liquid waste collection vessel 18 collects the culture solution S1 or the wash solution S2, which is stored in the culture vessel 12.

The culture vessel 12 is a vessel for accommodating the culture solution S1 containing the cells C. A suction nozzle 40 and a supply nozzle 42 are provided in the culture vessel 12.

The suction nozzle 40 has a suction port 40a through which the culture solution S1 (or the wash solution S2) in the culture vessel 12 is sucked. In the case of this embodiment, the suction nozzle 40 is provided so as to extend along a side wall portion 12a of the culture vessel 12. The suction port 40a is located in the vicinity of a bottom surface 12b inside the culture vessel 12.

Further, the suction nozzle 40 is connected to the pump 22. The pump 22 is, for example, a roller pump, and sucks the culture solution S1 (or the wash solution S2) in the culture vessel 12 via the suction nozzle 40. Further, the pump 22 feeds the sucked culture solution S1 (or the wash solution S2) to the liquid waste collection vessel 18 or the hollow fiber membrane filter 20. The on-off valve 28 is provided between the pump 22 and the liquid waste collection vessel 18, and the on-off valve 30 is provided between the pump 22 and the hollow fiber membrane filter 20. Those on-off valves 28 and 30 enable the pump 22 to selectively perform feeding of the culture solution S1 stored in the culture vessel 12 to the hollow fiber membrane filter 20 and feeding of the culture solution S1 (or the wash solution S2) stored in the culture vessel 12 to the liquid waste collection vessel 18.

The supply nozzle 42 has a supply port 42a through which the culture solution S1 (or the wash solution S2) is supplied into the culture vessel 12. In the case of this embodiment, the supply nozzle 42 is provided so as to extend along the side wall portion 12a of the culture vessel 12. The supply port 42a is located in the vicinity of the bottom surface 12b inside the culture vessel 12. In the case of this embodiment, the suction nozzle 40 and the supply nozzle 42 are arranged at a maximum distance from each other inside the culture vessel 12 so as to be opposed to each other.

Further, the supply nozzle 42 is connected to the culture solution supply vessel 14 and the wash solution supply vessel 16 through intermediation of the pump 24. The pump 24 is, for example, a roller pump, and supplies the culture solution S1 stored in the culture solution supply vessel 14 or the wash solution S2 stored in the wash solution supply vessel 16 into the culture vessel 12 via the supply nozzle 42. The on-off valve 32 is provided between the pump 24 and the culture solution supply vessel 14, and the on-off valve 34 is provided between the pump 24 and the wash solution supply vessel 16. Those on-off valves 32 and 34 enable the pump 24 to selectively perform supply of the culture solution S 1 stored in the culture solution supply vessel 14 to the culture vessel 12 and supply of the wash solution S2 stored in the wash solution supply vessel 16 to the culture vessel 12.

The hollow fiber membrane filter 20 is a filter configured to filter the culture solution S1 supplied from the pump 22. The filtered, that is, clarified culture solution S1 is returned into the culture vessel 12 via the supply nozzle 42. The unfiltered culture solution S1 is sent to the liquid waste collection vessel 18 via the pump 26.

Further, the culture apparatus 10 includes a tilting device 50 configured to enable tilting of the culture vessel 12 in a plurality of different directions in top view (as viewed from Z axis direction). This function allows the tilting device 50 to be used as an agitation device that agitates the culture solution S1 in the culture vessel 12.

FIG. 2 is a schematic partial sectional view of the tilting device in the culture apparatus. Further, FIG. 3 is a schematic partial sectional view of a part of the tilting device as viewed from a different direction. Moreover, FIG. 4 is a schematic partial sectional view of the tilting device in a state in which a stage is tilted.

As illustrated in FIG. 3 and FIG. 4, the tilting device 50 includes a stage 52 on which the culture vessel 12 is to be placed, and a rotary actuator 56 including a rotary table 54 which rotates about a rotation center axis R0 extending in the vertical direction (Z axis direction).

The stage 52 and the rotary actuator 56 are drivingly coupled to each other via a rocking head 58 and a tilting mechanism 60.

The rocking head 58 is provided in the tilting device 50 so as to support the stage 52 and to be rockable about a rocking axis R1 extending in the horizontal direction (X axis direction) and a rocking axis R2 extending in the horizontal direction (Y axis direction) and being orthogonal to the rocking axis R1. Further, the rocking head 58 includes, in a lower portion thereof, a coupling shaft 62 to be drivingly coupled to the rotary actuator 56 via the tilting mechanism 60. When the stage 52 takes a horizontal posture, the coupling shaft 62 of the rocking head 58 extends in the vertical direction (Z axis direction).

The tilting mechanism 60 is a link mechanism for tilting the stage 52 via the rocking head 58, that is, tilting the culture vessel 12 on the stage 52 with respect to the horizontal direction. Thus, the tilting mechanism 60 includes a base portion 64, a rocking head coupling portion 66 coupled to the rocking head 58, and link arms 68 for coupling the base portion 64 and the rocking head coupling portion 66 to each other.

The base portion 64 of the tilting mechanism 60 is mounted to the rotary table 54 of the rotary actuator 56. Thus, when the rotary actuator 56 is driven, the base portion 64 rotates together with the rotary table 54 about the rotation center axis R0.

The rocking head coupling portion 66 of the tilting mechanism 60 is inserted over the coupling shaft 62 of the rocking head 58 so as to be slidable through intermediation of, for example, a bearing or the like.

The link arms 68 of the tilting mechanism 60 are configured to couple the base portion 64 and the rocking head coupling portion 66 to each other. Specifically, each of the link arms 68 includes one end turnably fixed to the rocking head coupling portion 66, and another end turnably fixed to the base portion 64. A turning axis R3 at the one end of each of the link arms 68 and a rotation axis R4 at the another end thereof each extend in the horizontal direction, and are parallel to each other.

The rotary actuator 56 having the base portion 64 of the tilting mechanism 60 mounted thereon is raised and lowered in the vertical direction (Z axis direction) by a ball screw mechanism 70.

The ball screw mechanism 70 includes a screw shaft 72 extending in the vertical direction (Z axis direction), a nut 74 engaged with the screw shaft 72, and a motor 76 for rotating the screw shaft 72. The nut 74 is mounted to a raising/lowering bracket 78. The rotary actuator 56 is mounted to this raising/lowering bracket 78.

When the motor 76 of the ball screw mechanism 70 is driven, the rotary actuator 56 is raised and lowered together with the raising/lowering bracket 78 via the screw shaft 72 and the nut 74. For example, as illustrated in FIG. 4, when the rotary actuator 56 is raised by the ball screw mechanism 70, the stage 52 is tilted via the tilting mechanism 60. Specifically, the base portion 64 of the tilting mechanism 60 mounted to the rotary actuator 56 is raised so that the link arms 68 push the rocking head coupling portion 66. Thus, the rocking head 58 rotates together with the rocking head coupling portion 66 about at least one of the rocking axis R1 or the rocking axis R2 (rocking axis R2 in FIG. 5). In this manner, the stage 52 is tilted, and the culture vessel 12 on this stage 52 is also tilted. As a result, the culture vessel 12 is tilted with respect to the horizontal direction.

As illustrated in FIG. 4, when the rotary actuator 56 is driven under a state in which the stage 52 is tilted and thus the rotary table 54 is rotated, the tilting mechanism 60 rotates about the rotation center axis R0 so that a tilt direction of the stage 52 in top view (as viewed in Z axis direction), that is, a tilt direction TD of the culture vessel 12 changes. As a result, the culture solution S1 in the culture vessel 12 is agitated and the cells C in the culture solution S1 are cultured.

In such a tilting device 50, even when the rotary actuator 56 rotates the tilting mechanism 60, for example, one revolution, the stage 52 itself does not rotate, and only the tilt direction of the stage 52 is rotated one revolution instead. That is, a lowest part of the bottom surface 12b of the culture vessel 12 on the stage 52 is only sequentially changed to another part so as to circle around in top view (as viewed in Z axis direction). As a result, the culture solution S1 in the culture vessel 12 circles around to be agitated.

With such a tilting device 50, the expansion culture can be executed through use of only one culture vessel 12. Specifically, when agitating conditions (tilt angle θ of the stage 52, and rotation angle range and rotation speed of the rotary actuator 56) are changed depending on a liquid amount of the culture solution S1 in the culture vessel 12, culture solutions S1 in a small amount to a large amount can be agitated with agitating conditions suitable for cell growth.

The configuration of the culture apparatus 10 has been described so far. Now, an operation of the culture apparatus 10, in particular, replacement of the culture solution S1 in the culture vessel 12 is described.

FIG. 5 is a block diagram for illustrating a control system of the culture apparatus, which is associated with the replacement of the culture solution in the culture vessel.

As illustrated in FIG. 5, the culture apparatus 10 includes a controller 80 and a weight sensor 82. The controller 80 controls the plurality of pumps 22, 24, and 26, the plurality of on-off valves 28, 30, 32, 34, and 36, and the tilting device 50 (the rotary actuator 56 and the motor 76).

The controller 80 includes, for example, a processor such as a CPU or an MPU, a storage device such as a memory, and a circuit. The storage device stores a program that causes the processor to execute various operations. The circuit electrically connects the processor and devices external to the controller 80, such as the plurality of pumps 22 to 26, the plurality of on-off valves 28 to 36, and the tilting device 50, to each other.

Further, the controller 80 controls the plurality of pumps 22, 24, and 26, the plurality of on-off valves 28, 30, 32, 34, and 36, and the tilting device 50 based on, for example, a cell culture schedule that has been created by a user and stored in the storage device to thereby execute the culture of the cells C. The cell culture schedule includes a replacement timing for the culture solution S1 in the culture vessel 12. The controller 80 executes the replacement of the culture solution S1 in the culture vessel 12 during the culture of the cells C in accordance with the cell culture schedule.

The weight sensor 82 is a sensor configured to measure weight of the culture vessel 12. A role of the weight sensor 82 is described later.

At the replacement timing for the culture solution S1 during the culture of the cells C, the controller 80 starts executing the following control. In the case of this embodiment, the replacement of the culture solution S1 is performed by collecting almost all the culture solution S1 stored in the culture vessel 12 into the liquid waste collection vessel 18 and then supplying the fresh culture solution S1 stored in the culture solution supply vessel 14 into the culture vessel 12.

FIG. 6A to FIG. 10A are top views of the culture vessel during the replacement of the culture solution. Further, FIG. 6B to FIG. 10B are sectional views of the culture vessel during the replacement of the culture solution. FIG. 6B to FIG. 10B are sectional views taken along the tilt direction TD of the culture vessel 12 and the vertical direction (Z axis direction).

As illustrated in FIG. 6A and FIG. 6B, at the replacement timing for the culture solution S1, the controller 80 starts controlling the motor 76 for the tilting device 50 to tilt the culture vessel 12 by a predetermined angle θ with respect to the horizontal direction so that the culture solution S1 has the largest liquid depth at a position far from a suction range SR for the suction port 40a of the suction nozzle 40. The cell vessel 12 is tilted as described above so as to cause the cells C1 floating in the culture solution S1 to be precipitated in a corner portion defined by the side wall portion 12a and the bottom surface 12b of the culture vessel 12, which is at the position far from the suction range SR for the suction port 40a of the suction nozzle 40. The predetermined angle θ is determined by elapsed time from start of the culture, that is, the number of cells in the culture vessel 12. Further, a speed at which the culture vessel 12 is tilted by the predetermined angle θ with respect to the horizontal direction is set so as not to apply stress to the cells. For example, the culture vessel 12 is only required to be tilted at a speed that does not cause a liquid surface of the culture solution S1 to be ruffled so that stress applied to the cells is reduced. Further, an ability of the weight sensor 82 to detect waves on the liquid surface of the culture solution S1 may also be used. When the weight sensor 82 detects that fluctuations equal to or larger than a predetermined ratio with respect to a total weight at a predetermined frequency or lower for a weight value, the speed at which the culture vessel 12 is tilted is only required to be decreased so as to reduce stress on the cells. Further, the speed at which the culture vessel 12 is tilted may be set so that a Reynolds number becomes equal to or smaller than a predetermined value, for example, 2,300 or smaller.

The suction range SR is a range in which the culture solution S1 can be sucked through the suction nozzle 40 and is determined depending on a size of the suction port 40a of the suction nozzle 40 and power of the pump 22.

In the case of this embodiment, the tilting device 50 tilts the culture vessel 12 in the tilt direction TD so that the culture solution S1 has the largest liquid depth at the position far from the suction range SR for the suction port 40a of the suction nozzle 40. As a result, the cells C are precipitated in the vicinity of the supply nozzle 42 that is opposed to the suction nozzle 40 located at the maximum distance therefrom.

As illustrated in FIG. 6A and FIG. 6B, after the cells C are sufficiently precipitated at the location at which the culture solution S1 has the largest liquid depth, suction of the culture solution S1 stored in the culture vessel 12 through the suction nozzle 40 is started. Specifically, the controller 80 brings the on-off valve 30 into a closed state while bringing the on-off valve 28 into an open state and actuates the pump 22. As a result, the culture solution S1 in the culture vessel 12 is moved into the liquid waste collection vessel 18. At this time, the culture solution S1 around the cells C in a precipitated state is away from the suction port 40a of the suction nozzle 40, and thus does not substantially flow. As a result, stress is not substantially applied from the culture solution S1 on the cells C.

When the culture solution S1 stored in the culture vessel 12 is sucked through the suction nozzle 40, the culture solution S1 in the culture vessel 12 is naturally decreased to thereby expose the suction port 40a of the suction nozzle 40 above a liquid level FL of the culture solution S1. As a result, the culture solution S1 cannot be sucked through the suction nozzle 40 anymore. In order to cope with this inconvenience, as illustrated in FIG. 7A to FIG. 9A and FIG. 7B to FIG. 9B, the tilting device 50 changes the tilt direction TD of the culture vessel 12 as the culture solution S1 stored in the culture vessel 12 is being decreased due to the suction through the suction nozzle 40. More specifically, the tilting device 50 changes the tilt direction TD of the culture vessel 12 so that the suction port 40a remains positioned below the liquid level FL of the culture solution S1 that continuously falls due to the suction through the suction nozzle 40.

In the case of this embodiment, the weight sensor 82 measures weight of the culture vessel 12. Weight of the culture solution S1 can be obtained by subtracting the weight of the culture vessel 12 from the result of measurement performed by the weight sensor 82. An accommodation amount of the culture solution S1 in the culture vessel 12 can be obtained from the calculated weight. A height position of the liquid level FL of the culture solution S1 in the culture vessel 12 can be obtained from the accommodation amount of the culture solution S1, an internal shape of the culture vessel 12, and the tilt angle θ of the culture vessel 12. When the height position of the liquid level FL is obtained, the tilt direction TD of the culture vessel 12, which is required to position the suction port 40a of the suction nozzle 40 below the liquid level FL, can be obtained. In other words, when the suction port 40a of the suction nozzle 40 remains positioned below the liquid level FL of the culture solution S1 stored in the culture vessel 12 that continuously falls due to the suction through the suction nozzle 40, the result of measurement performed by the weight sensor 82 and the tilt direction TD of the culture vessel 12 have a correlative relationship.

Thus, the controller 80 controls the tilting device 50 (the rotary actuator 56 thereof) based on the result of measurement performed by the weight sensor 82. Thus, as illustrated in FIG. 7A to FIG. 9A and FIG. 7B to FIG. 9B, the tilting device 50 changes the tilt direction TD of the culture vessel 12 so that the suction port 40a remains positioned below the liquid level FL of the culture solution S1 that continuously falls due to the suction through the suction nozzle 40. In other words, the tilt direction TD of the culture vessel 12 is changed so that the cells C precipitated at the position at which the culture solution S1 has the largest liquid depth are moved closer to the suction nozzle 40.

When the culture solution S1 in the culture vessel 12 is collected through the suction nozzle 40 as described above, stress applied on the cells C due to flow of the culture solution S1 is reduced in comparison to a case in which the cells C are suspended throughout the culture solution S1. Specifically, while the culture solution S1 is being sucked through the suction nozzle 40, the cells C are precipitated at the position as far as possible from the suction range SR for the suction port 40a of the suction nozzle 40, at which the culture solution S1 has the largest liquid depth, that is, at the position where there is substantially no flow of the culture solution S1. Thus, the cells C are less liable to be subjected to stress from the culture solution S1. As a result, damage to the cells C is reduced. Further, the cells C are less likely to be sucked through the suction nozzle 40, and thus the number of cells remaining in the culture vessel 12 increases.

After the collection of the culture solution S1 that has been stored in the culture vessel 12 into the liquid waste collection vessel 18 is completed, the controller 80 starts supply of the culture solution S1 stored in the culture solution supply vessel 14 into the culture vessel 12. First, the controller 80 brings the on-off valve 28 into a closed state and stops the pump 22. Subsequently, the controller 80 brings the on-off valve 32 into an open state and actuates the pump 24. Thus, the culture solution S1 stored in the culture solution supply vessel 14 is supplied into the culture vessel 12 via the supply nozzle 42.

When the culture solution S1 is supplied through the supply nozzle 42 into the culture vessel 12, as illustrated in FIG. 10A and FIG. 10B, the tilting device 50 tilts the culture vessel 12 so that the culture solution has the largest liquid depth at the position far from the supply port 42a of the supply nozzle 42. The cells C are precipitated at the position.

In the case of this embodiment, the tilting device 50 tilts the culture vessel 12 in the tilt direction TD so that the cells C are precipitated at the position "farthest" away from the supply port 42a of the supply nozzle 42. As a result, the cells C are precipitated in the vicinity of the suction nozzle 40 that is opposed to the supply nozzle 42 located at the maximum distance therefrom. In the case of this embodiment, as illustrated in FIG. 10A and FIG. 10B, the suction nozzle 40 and the supply nozzle 42 are arranged at the maximum distance from each other inside the culture vessel 12 so as to be opposed to each other. Thus, after the suction of the culture solution S1 stored in the culture vessel 12 through the suction nozzle 40 is completed, the supply port 42a of the supply nozzle 42 is located away from the precipitated cells C.

As illustrated in FIG. 10A and FIG. 10B, the culture solution S1 flowing out from the supply port 42a of the supply nozzle 42 flows on the bottom surface 12a of the culture vessel 12 to reach the precipitated cells C. Then, ultimately, the cells C are immersed in the culture solution S1 supplied from the supply nozzle 42. It is preferred that a supply speed at which the culture solution S1 is supplied from the supply port 42a of the supply nozzle 42 into the culture vessel 12 be such that the cells C are allowed to be maintained in a precipitated state in the culture solution S1. With this setting of the speed, stress applied to the cells C can be reduced.

When the culture solution S1 is supplied into the culture vessel 12 through the supply nozzle 42 as described above, stress from the culture solution S1 on the cells C can be reduced in comparison to a case in which the cells C are present in front of or in the vicinity of the supply port 42a of the supply nozzle 42.

After the supply of the culture solution S1 stored in the culture solution supply vessel 14 into the culture vessel 12 is completed, the controller 80 closes the on-off valve 32 and stops the pump 24. Thus, the replacement of the culture solution S1 in the culture vessel 12 is completed. Then, the controller 80 controls the tilting device 50 to restart the agitation of the culture solution S1 in the culture vessel 12.

The replacement of the culture solution S1 in the culture vessel 12 can also be performed by another method. More specifically, the culture solution S1 is supplied into the culture vessel 12 through the supply nozzle 42 while the culture solution S1 in the culture vessel 12 is being sucked through the suction nozzle 40. In this manner, the replacement of the culture solution S1 in the culture vessel 12 can be performed in a short period of time. In this case, as illustrated in FIG. 8A, the tilting device 50 tilts the culture vessel 12 so that a position inside the culture vessel 12, which is at an equal distance from the suction nozzle 40 and from the supply nozzle 42, is a position at which the culture solution has the largest liquid depth and the cells C are precipitated at the position. Thus, the suction of the culture solution S1 stored in the culture vessel 12 through the suction nozzle 40 and the supply of the culture solution S1 into the culture vessel 12 through the supply nozzle 42 can be simultaneously performed while stress on the cells C is reduced.

Further, also when liquid containing the culture solution S1 sucked through the suction nozzle 40 is filtered through the hollow fiber membrane filter 20 and the filtered liquid containing the culture solution S1 is returned to the culture vessel 12 through the supply nozzle 42, as illustrated in FIG. 8A, the tilting device 50 tilts the culture vessel 12 so that the position inside the culture vessel 12, which is at the largest distance from each of the suction nozzle 40 and the supply nozzle 42, is a position at which the culture solution has the largest liquid depth. The cells C are precipitated at the position. Thus, the culture solution S1 in the culture vessel 12 can be clarified while stress on the cells C is reduced.

In this case, the amount (supply amount) of the liquid containing the culture solution S1, which has been filtered through the hollow fiber membrane filter 20, to be returned to the culture vessel 12, may be adjusted based on the result of measurement performed by the weight sensor 82. More specifically, the supply amount of the liquid containing the culture solution S1, which has been filtered through the filter 20, into the culture vessel 12 may be adjusted so that a time rate of change of the measurement value obtained by the weight sensor 82 falls within a predetermined range, that is, the amount of the culture solution S1 stored in the culture vessel 12 does not rapidly change. In this manner, a large change in culture environment can be suppressed, and thus stress on the cells C can be reduced.

For washing of the cells C in the culture vessel 12 with the wash solution S2, after the culture solution S1 in the culture vessel 12 is collected by the above-mentioned method illustrated in FIG. 6A to FIG. 10A and FIG. 6B to FIG. 10B, the wash solution S2 in the wash solution supply vessel 16 is supplied into the culture vessel 12 via the supply nozzle 42. In this manner, the culture solution S1 in the culture vessel 12 is replaced by the wash solution S2. Also in this case, the tilting device 50 tilts the culture vessel so that the culture solution has the largest liquid depth at a position far from the supply port 42a of the supply nozzle 42, and the cells C are precipitated at the position.

Further, the culture apparatus 10 also has a function to suck an adequate amount of the culture solution S1 from the culture vessel 12 through the suction nozzle 40 after supplying the culture solution S1 into the culture vessel 12 through the supply nozzle 42. With this function, the cells C can be brought into contact with the fresh culture solution S1 while the amount of the old culture solution S1 in the culture vessel 12 is being reduced without a large reduction in the amount of the culture solution S1 with which the cells C in the culture vessel 12 are in contact.

Further, the culture apparatus 10 also has a function to increase, decrease, or keep constant the amount of the culture solution S1 in the culture vessel 12 while diluting the old culture solution S1 with the fresh culture solution S1 through the adjustment of the supply amount of the culture solution S1 into the culture vessel 12 and the suction amount of the culture solution S1 sucked from the culture vessel 12. With this function, control of the amount of the culture solution S 1 and the dilution thereof in accordance with a state of the cells C in the culture vessel 12 can be achieved.

According to this embodiment described above, in the cell culture for culturing the cells in the culture vessel with the culture solution, stress on the cells, which is caused when the culture solution is collected from the culture vessel or when the culture solution is supplied into the culture vessel, can be reduced.

In culture medium replacement, which is performed also in this embodiment, dilution at a super high rate is performed as in an operation of sucking liquid separated from the cells through centrifugal separation and seeding the cells in a fresh culture liquid. With the culture apparatus according to this embodiment, the centrifugal separation cannot be performed in the culture medium replacement for the culture solution in the vessel. Thus, a precipitation property of the cells is used for the culture medium replacement. The dilution at a low rate is less efficient than the centrifugal separation. However, the above-mentioned culture medium replacement operation is effective in the culture medium replacement for a large amount of cells to be cultured with single-use vessels. For example, when replacement of 10 liters of culture solution is to be performed and it is supposed that 10-fold dilution allows cells to be cultured without being affected by an old culture solution, 100 liters of fresh culture solution is required for general dilution and culture for which the centrifugal separation cannot be used. In such a case, a large amount of culture solution, which is expensive, is required. Meanwhile, with the culture apparatus according to this embodiment, the old culture solution is reduced after the cells are precipitated. Thus, the amount of the fresh culture solution for diluting the culture solution by 10 times can be reduced, and hence the culture apparatus is extremely effective. Further, with the culture apparatus according to this embodiment, the suction amount of the culture solution can be set based on a liquid amount calculated from the result of measurement performed by the weight sensor and a specific gravity of the liquid. Thus, suction of an appropriate amount of the old culture solution and supply of an appropriate amount of the fresh culture solution for culture medium replacement can be performed.

The present disclosure has been described above by giving the embodiment described above, but the embodiment of the present disclosure is not limited to those.

For example, in the case of the first embodiment described above, one suction nozzle 40 and one supply nozzle 42 are provided in the culture vessel 12. However, the suction nozzle and the supply nozzle according to the embodiment of the present disclosure are not limited to those described above.

FIG. 11 is a schematic view for illustrating a configuration of a culture apparatus according to another embodiment of the present disclosure.

As illustrated in FIG. 11, in a culture apparatus 110 according to another embodiment, a plurality of suction nozzles 140A to 140C and a plurality of supply nozzles 142A to 142C are provided in a culture vessel 12.

The plurality of suction nozzles 140A to 140C are connected to a pump 22. On-off valves 144A to 144C are arranged between the plurality of suction nozzles 140A to 140C and the pump 22, respectively. Further, distances from suction ports 140a of the plurality of suction nozzles 140A to 140C to a bottom surface 12a of the culture vessel 12 are different from each other.

The plurality of supply nozzles 142A to 142C are connected to a pump 24. On-off valves 146A to 146C are arranged between the plurality of supply nozzles 142A to 142C and the pump 24, respectively. Further, distances from supply ports 142a of the plurality of supply nozzles 142A to 142C to the bottom surface 12a of the culture vessel 12 are different from each other.

The plurality of suction nozzles 140A to 140C and the plurality of supply nozzles 142A to 142C described above allow replacement of a culture solution S1 in the culture vessel 12 by various methods. For example, while the culture solution S1 is being sucked through any of the suction nozzles, each with the suction port 140a positioned below a liquid level FL of the culture solution S1, the culture solution S1 can be supplied into the culture vessel 12 through any of the supply nozzles, each with the supply port 142a exposed from the culture solution S1. Specifically, an optimal replacement method for the culture solution S1 in accordance with the kind of cells C can be put into practice.

Further, in the case of the embodiments described above, as illustrated in FIG. 2 to FIG. 4, the tilting device 50 can tilt the culture vessel 12 in a plurality of different directions in top view by using the rotary actuator 56 and the motor 76 as drive sources and adopting the link mechanism. However, the tilting device according to the embodiments of the present disclosure is not limited to that described above. The tilting device of the culture apparatus according to the embodiments of the present disclosure may have any structure as long as the tilting device can tilt the culture vessel in a plurality of different directions in top view.

Further, in the case of the embodiments described above, the liquid amount of the culture solution S1 in the culture vessel 12 is obtained based on the result of measurement performed by the weight sensor 82. However, a way of obtaining the liquid amount according to the embodiments of the present disclosure is not limited to that described above. For example, the liquid amount of the culture solution S1 in the culture vessel 12 can be obtained from a cumulative supply amount of the culture solution supplied through the supply nozzle and a cumulative collection amount of the culture solution collected through the suction nozzle. Each of the cumulative supply amount and the cumulative collection amount can be calculated from drive time of each of the pumps and a feed amount per unit time from each of the pumps.

Specifically, the culture apparatus according to the embodiments of the present disclosure includes, in a broad sense, the culture vessel configured to accommodate the culture solution for culturing the cells, the tilting device configured to enable tilting of the culture vessel in the plurality of different directions in top view, and the suction nozzle having the suction port through which the culture solution in the culture vessel is sucked, the suction port being arranged inside the culture vessel. When the culture solution in the culture vessel is sucked through the suction nozzle, the tilting device tilts the culture vessel so that the culture liquid has the largest liquid depth at the position far from the suction range for the suction port of the suction nozzle.

Further, the culture apparatus according to the embodiments of the present disclosure includes, in a broad sense, the culture vessel configured to accommodate the culture solution for culturing the cells, the tilting device configured to enable tilting of the culture vessel in the plurality of different directions in top view, and the supply nozzle having the supply port through which the culture solution is supplied into the culture vessel, the supply port being arranged inside the culture vessel. When the culture solution is supplied into the culture vessel through the supply nozzle, the tilting device tilts the culture vessel so that the culture liquid has the largest liquid depth at the position far from the supply port of the supply nozzle.

### [Industrial Applicability]

The present disclosure is applicable to cell culture for culturing cells while a culture solution is agitated.

## Claims

1. A culture apparatus comprising:
a culture vessel configured to accommodate a culture solution for culturing cells;
a tilting device configured to enable tilting of the culture vessel in a plurality of different directions in top view; and
a suction nozzle having a suction port through which the culture solution in the culture vessel is sucked, the suction port being arranged inside the culture vessel,
wherein, the tilting device is configured to, when the culture solution in the culture vessel is sucked through the suction nozzle, tilt the culture vessel so that the culture solution has the largest liquid depth at a position far from a suction range for the suction port of the suction nozzle.

2. The culture apparatus according to claim 1, wherein the tilting device is configured to change a tilt direction of the culture vessel so that the suction port remains positioned below a liquid level of the culture solution, the liquid level continuously falling due to suction through the suction nozzle.

3. The culture apparatus according to claim 2, further comprising a weight sensor configured to measure weight of the culture vessel,
wherein the tilting device is configured to change the tilt direction of the culture vessel based on a result of measurement performed by the weight sensor.

4. The culture apparatus according to claim 1, further comprising a supply nozzle having a supply port through which a culture solution is supplied into the culture vessel, the supply port being arranged inside the culture vessel,
wherein the tilting device is configured to, when the culture solution is supplied into the culture vessel through the supply nozzle, tilt the culture vessel so that the culture solution has the largest liquid depth at a position far from the supply port of the supply nozzle.

5. The culture apparatus according to claim 4, wherein, after the culture solution in the culture vessel is sucked through the suction nozzle, the culture solution is supplied into the suction vessel through the supply nozzle.

6. The culture apparatus according to claim 4,
wherein the tilting device is configured to tilt the culture vessel so that the culture solution has the largest liquid depth at a position inside the culture vessel, the position being the farthest from each of the suction nozzle and the supply nozzle, and
wherein the culture solution is supplied into the culture vessel through the supply nozzle while the culture solution in the culture vessel is being sucked through the suction nozzle.

7. The culture apparatus according to claim 6, further comprising a filter configured to filter only the culture solution sucked through the suction nozzle,
wherein liquid containing the culture solution filtered through the filter is supplied into the culture vessel from the supply nozzle.

8. The culture apparatus according to claim 7, further comprising a weight sensor configured to measure weight of the culture vessel,
wherein a supply amount of the liquid containing the culture solution filtered through the filter into the culture vessel is adjusted so that a time rate of change of a measurement value obtained by the weight sensor falls within a predetermined range.

9. The culture apparatus according to claim 1,
wherein the tilting device includes:
a stage on which the culture vessel is to be placed;
a rotary actuator including a rotary table configured to rotate about a rotation center axis extending in a vertical direction;
a rocking head configured to support the stage and to be rockable about a first rocking axis extending in a horizontal direction and a second rocking axis extending in the horizontal direction and being orthogonal to the first rocking axis, the rocking head including a coupling shaft;
a tilting mechanism including a rocking head coupling portion, which is slidably inserted over the coupling shaft of the rocking head to be coupled to the rocking head, a base portion mounted to the rotary table of the rotary actuator, and a link arm including one end turnably fixed to the rocking head coupling portion and another end turnably fixed to the base portion; and
a rotary actuator raising/lowering mechanism configured to raise and lower the rotary actuator in the vertical direction.

10. A culture apparatus comprising:
a culture vessel configured to accommodate a culture solution for culturing cells;
a tilting device configured to enable tilting of the culture vessel in a plurality of different directions in top view; and
a supply nozzle having a supply port through which the culture solution is supplied into the culture vessel, the supply port being arranged inside the culture vessel,
wherein, the tilting device is configured to, when the culture solution is supplied into the culture vessel through the supply nozzle, tilt the culture vessel so that the culture solution has the largest liquid depth at a position far from the supply port of the supply nozzle.
